# EUROPÄISCHE PATENTSCHRIFT

(11) **EP 0 847 382 B1**
(45) Veröffentlichungstag und Bekanntmachung des Hinweises auf die Patenterteilung: **20.12.2000**
(21) Anmeldenummer: 96930052.4
(22) Anmeldetag: 19.08.1996
(51) Int. Cl.: C07C 229/56

(54) **MONOESTER DER CARBOXYMETHYLENANTHRANILSÄUREN UND VERFAHREN ZU IHRER HERSTELLUNG**
MONOESTERS OF CARBOXYMETHYLENE ANTHRANILIC ACIDS AND PROCESS FOR PREPARING THE SAME
MONOESTERS DES ACIDES CARBOXYMETHYLENANTHRANILIQUES ET LEUR PROCEDE DE PREPARATION

(30) Priorität: 31.08.1995 DE 19532053; 14.05.1996 DE 19619395
(43) Veröffentlichungstag der Anmeldung: 17.06.1998
(73) Patentinhaber: Clariant GmbH, 65929 Frankfurt am Main (DE)
(72) Erfinder: PAPENFUHS, Theodor, D-60433 Frankfurt am Main (DE); PFIRMANN, Ralf, D-64347 Griesheim (DE); KRAUSE, Stefan, D-65929 Frankfurt (DE); NEUMANN-GRIMM, Doris, D-60388 Frankfurt am Main (DE)
(86) Internationale Anmeldenummer: EP9603631
(87) Internationale Veröffentlichungsnummer: WO9708131

(56) Entgegenhaltungen:
- EP-A- 0 218 999
- EP-A- 0 360 417
- J. HETEROCYCL. CHEM. (JHTCAD,0022152X);77; VOL.14 (7); PP.1139-43, UNIV. PATRAS;LAB. ORG. CHEM.; PATRAS; GREECE, XP002021535 STAVROPOULOS G ET AL: "Synthesis of 4-substituted 1,4-benzodiazepine-3,5-diones"
- CHEMICAL ABSTRACTS, vol. 108, no. 25, 20.Juni 1988 Columbus, Ohio, US; abstract no. 221379, HENNING H G ET AL: "Syntheses of heterocycles from 5-phenylisoxazolium salts. 1. Synthesis and thermal behavior of.beta.-keto imides" XP002021536 & Z. CHEM. (ZECEAL,00442402);87; VOL.27 (8); PP.290-2, HUMBOLDT-UNIV. BERLIN;SEKT. CHEM.; BERLIN; DDR-1040; GER. DEM. REP. (DD),

## Beschreibung

Die Erfindung betrifft Monoester der Carboxymethylen-4-chloranthranilsäure und Verfahren zu ihrer Herstellung.

In der Literatur sind derartige Monoester der Carboxymethylen-4-chloranthranilsäure noch nicht beschrieben. Derartige Verbindungen sind wertvolle Ausgangsprodukte für neuartige Synthesevarianten zur Herstellung von Aldose-Reduktase-Hemmern - wie aus der am gleichen Tag wie die vorliegende deutsche Patentanmeldung (Aktenzeichen 195 32 053.0) eingereichten deutschen Patentanmeldung (Aktenzeichen: 195 32 052.2) hervorgeht - und machen neue Verbindungsklassen wie z.B. Diester der Carboxymethylen-4-chloranthranilsäure mit verschiedenen Esterresten erstmals zugänglich.

Der von 2,4-Dichlorbenzoesäure ausgehende, recht einfache und kurze Syntheseweg, der zu den als Ausgangsstoffe für die Herstellung von Aldose-Reduktase-Hemmern verwendeten Chinazolindionen der Formel (A) führt, ist nachfolgend schematisch vereinfacht wiedergegeben:

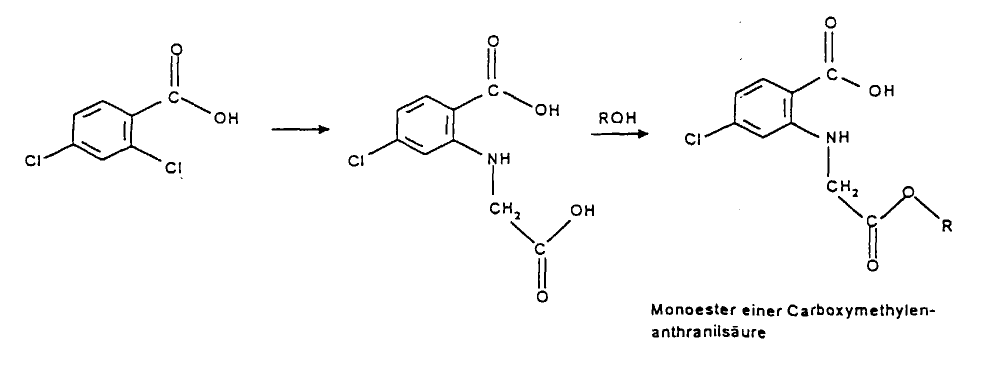

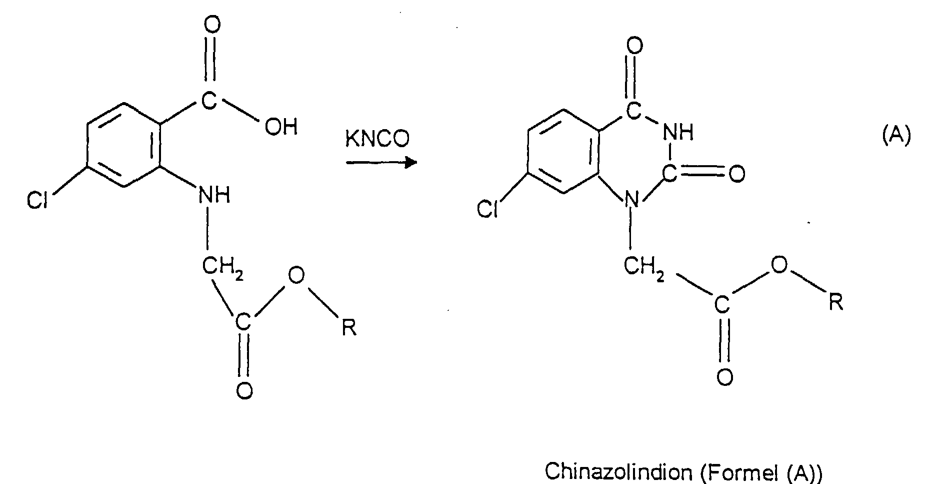

Bei dem vorstehend genannten Chinazolindion (Formel (A)) handelt es sich um einen Ester der 1,2,3,4-Tetrahydro-2,4-dioxochinazolin-1-ylessigsäure, der gemäß EP 218 999 als Ausgangsmaterial für die Herstellung von Aldose-Reduktase-Hemmern verwendet werden kann.

Um die Möglichkeiten der Synthese von Aldose-Reduktase Hemmern zu erweitern und neue Verbindungen zugänglich zu machen, bestand daher ein Bedarf, derartige Verbindungen bereitzustellen.

Diese Aufgabe wird gelöst durch Verbindungen der Formel (I) 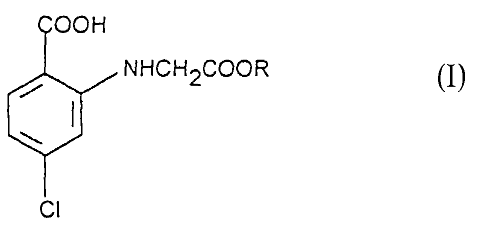 worin R für ein geradkettiges oder verzweigtes (C₁-C₂₀)-Alkyl, Phenyl oder CH₂-Phenyl, wobei die Alkylgruppe oder Phenylgruppe mit Halogen, (C₁-C₄)-Alkyl, (C₁-C₄)-Alkoxy, insbesondere die Alkylgruppe mit Halogen oder (C₁-C₄)-Alkoxy und insbesondere die Phenylgruppe mit (C₁-C₄)-Alkyl oder (C₁-C₄)-Alkoxy substituiert sein kann, steht.

In den Verbindungen der Formel (I) steht R insbesondere für ein geradkettiges oder verzweigtes (C₁-C₁₂)-Alkyl, Phenyl oder CH₂-Phenyl, bevorzugt für ein geradkettiges oder verzweigtes (C₁-C₆)-Alkyl oder Phenyl. Der Vollständigkeit halber sei an dieser Stelle erwähnt, daß es sich bei den Verbindungen der Formel (I) um Monoester der N-Carboxymethylen-4-chloranthranilsäure handelt. Von besonderem Interesse ist N-Carbethoxymethylen-4-chloranthranilsäure und N-Carbmethoxymethylen-4-chloranthranilsäure, N-Carbisopropoxymethylen-4-chloranthranilsäure, N-Carbpropoxymethylen-4-chloranthranilsäure, N-Carbbutoxy-methylen-4-chloranthranilsäure, N-Carbhexoxymethylen-4-chloranthranilsäure und N-Carbbenzoxymethylen-4-chloranthranilsäure.

Die Erfindung betrifft ferner ein Verfahren zur Herstellung der Verbindungen der Formel (I), dadurch gekennzeichnet, daß N-Carboxymethylen-4-chloranthranilsäure mit Alkoholen ROH, in Gegenwart eines Katalysators und gegebenenfalls eines Lösungsmittels, umgesetzt wird. Die bei der Herstellung der Verbindungen der Formel (I) eingesetzte N-Carboxymethylen-4-chloranthranilsäure ist durch die Formel (II) 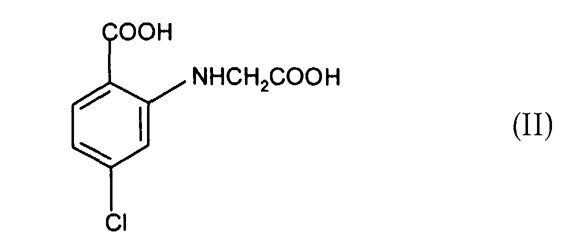 beschrieben. Der Rest R im Alkohol ROH hat die in der Formel (I) bereits genannte Bedeutung.

Die Umsetzung kann in An- oder Abwesenheit eines Lösungsmittels erfolgen. Als Lösungsmittel können aliphatische oder aromatische Kohlenwasserstoffe, z.B. Cyclohexan, Toluol, Benzol, der Alkohol ROH oder Gemische davon dienen. Der Alkohol ROH wird in einem Überschuß von 1 bis 1000 Mol, vorteilhaft 1,5 bis 500 Mol, bevorzugt 2 bis 200 Mol pro Mol N-Carboxymethylen-4-chloranthranilsäure eingesetzt.

Als Katalysator eignen sich wasserentziehende Mittel wie Carbonyldiimide, starke anorganische oder organische Säuren oder deren Salze oder Lewissäuren. Vielfach bewährt haben sich z.B. Dicyclohexylcarbodiimid, N,N-Carbonyl-diimidazol, p-Toluolsulfonsäure, Pyridinium-p-toluolsulfonat, Trifluoressigsäure, Bortrifluorid, Natriumsulfat, Schwefelsäure oder Salzsäure, insbesondere Schwefelsäure oder Salzsäure, bevorzugt Schwefelsäure. Analog können Mischungen der genannten Reagenzien eingesetzt werden. Die Konzentration des Katalysators kann zwischen 0,5 und 100 Mol-% liegen, vorteilhaft zwischen 1 und 50 Mol-%, bevorzugt 2 bis 30 Mol-%, bezogen auf N-Carboxymethylen-4-chloranthranilsäure.

Das Verfahren gestaltet sich besonders einfach, wenn man 2,5 bis 15 Mol-% Katalysator, bezogen auf eingesetzte N-Carboxymethylen-4-chloranthranilsäure einsetzt.

Nach einer bevorzugten Variante setzt man 5 bis 15 Mol-% Schwefelsäure in Form von konzentrierter Schwefelsäure je Mol Carboxymethylen-4-chloranthranilsäure als Katalysator ein.

Man führt die Umsetzung üblicherweise bei 20 bis 200, insbesondere 40 bis 160, bevorzugt 50 bis 120°C durch. Diese Temperaturen können ein Arbeiten unter Druck erforderlich machen. Dies ist der Fall, wenn die Reaktionstemperatur oberhalb der Siedetemperatur der jeweiligen Reaktionsmischung liegt.

Die Reaktionstemperaturen liegen zwischen 20°C und der Siedetemperatur des jeweiligen Alkohols, vorteilhaft zwischen 40°C und der jeweiligen Siedetemperatur, bevorzugt zwischen 60°C und der jeweiligen Siedetemperatur. Bei dieser Verfahrensweise kann man unter Normaldruck arbeiten.

Die Reaktionszeiten betragen 0,5 bis 60 h, vorteilhaft 0,75 bis 30 h, bevorzugt 1 bis 20 h.

Nach einer besonderen Verfahrensvariante kann das Lösungsmittel auch als Schleppmittel zur Abtrennung des bei der Veresterung entstehenden Wassers, beispielsweise durch Azeotropdestillation, dienen. Hierzu lassen sich aliphatische und aromatische Kohlenwasserstoffe, beispielsweise n-Hexan, Cyclohexan, Toluol, Xylol, aliphatische oder cycloaliphatische Ketone, beispielsweise n-Butanon, 4-Methyl-2-pentanon, Cyclohexanon, aber auch einfach oder mehrfach chlorierte aliphatische oder aromatische Kohlenwasserstoffe, beispielsweise Methylenchlorid, Chloroform, Chlorbenzol verwenden. Besonders geeignet ist Chloroform.
Eine weitere Möglichkeit, das bei der Veresterung enstehende Wasser zu entfernen, besteht darin, ein wasserbindendes Mittel, beispielsweise einen Orthoester, insbesondere Orthoameisensäuretrimethylester oder Orthoameisensäuretriethylester zuzusetzen. Besonders günstig kann es sein, die Veresterung bis zu einem bestimmten Umsatz, beispielsweise 50 bis 90 % oder auch mehr, insbesondere 55 bis 75 %, ablaufen zu lassen und anschließend einen Orthoester zur Vervollständigung der Umsetzung zuzusetzen.

Nach einer weiteren Verfahrensvariante läßt sich die Azeotropdestillation mit dem Zusatz eines wasserbindenden Mittels, insbesondere eines Orthoesters verbinden. Auch in diesem Falle kann es vorteilhaft sein, die Veresterung bis zu einem bestimmten Umsatz, beispielsweise 50 bis 90 % oder auch mehr, insbesondere 50 bis 75 %, mittels Azeotropdestillation ablaufen zu lassen und anschließend das wasserbindende Mittel, beispielsweise einen Orthoester zuzusetzen, um die Umsetzung zu vervollständigen.

Es ist besonders überraschend, daß unter diesen Reaktionsbedingungen nur Umsetzung zum Monoester eintritt, da in der Literatur nur Umsetzungen zum Diester beschrieben sind.

So wird in J. Med. Chem. 1991, 34, 1283, und J. Heterocycl. Chem. 1987, 24, 811 die Reaktion der Carboxymethylen-4-chloranthranilsäure mit Methanol und Schwefelsäure, in J. Prakt. Chem. 1929, 120, 64 mit Methanol und Salzsäure zum Diester beschrieben. In Hinblick hierauf ist es überraschend, daß unter Einsatz vergleichsweise geringer Mengen Katalysator ein Monoester entsteht, und es war ferner nicht zu erwarten, daß sich lediglich einer der beiden möglichen Monoester mit hoher Selektivität bildet.

Die beschriebenen Verfahrensschritte können unter Unter-, Über- oder Normaldruck durchgeführt werden.

Die folgenden Beispiele erläutern die Erfindung, ohne sie zu beschränken.

### Beispiel 1:

74,5 g (324,8 mmol) N-Carboxymethylen-4-chloranthranilsäure werden in 1660 g Ethanol suspendiert, mit 2,9 g (2,96 mmol) konz. Schwefelsäure versetzt und 10 h zum Sieden erhitzt. Nach 5 h Reaktionszeit wird nochmals die gleiche Menge Schwefelsäure zugesetzt. Nach 10 h wird das Ethanol abdestilliert, das ausfallende Produkt in der Kälte abfiltriert und mit kaltem Ethanol gewaschen. Man erhält 55,5 g (0,22 mol, 73 %) N-Carbethoxymethylen-4-chlor-anthranilsäure.
Schmelzpunkt: 166 bis 167°C
¹H-NMR (DMSO-d⁶): 1,22 (t,CH₃ Ethylester), 4,11-4,21 (m, 2 CH₂), 6,64 (dd, H-C(5)), 6,66-6,69 (m, H-C(3)), 7,80 (d, H-C(6)), 8,21-8,36 (NH) MS: 259, 257, 186, 184, 169, 168, 167, 166

Der entsprechende Diethylester hat sich, ausgewiesen durch HPLC-Analyse, lediglich in einer Menge < 0,2 % gebildet.

### Beispiel 2:

5 g (21,8 mmol) N-Carboxymethylen-4-chloranthranilsäure werden in 50 ml Methanol eingetragen und mit 222 mg (2,3 mmol) Schwefelsäure versetzt. Man erhitzt 4 h unter Rückfluß und kühlt anschließend auf Raumtemperatur ab. Das ausgefallene Produkt wird abgesaugt, mit Methanol gewaschen und getrocknet. Man erhält 4,0 g (16,4 mmol; 75 %) N-Carbmethoxymethylen-4-chlor-anthranilsäure.
Schmelzpunkt: 201 bis 203°C
MS: 245, 243, 186, 184, 168, 166

### Beispiel 3:

5 g (21,8 mmol) N-Carboxymethylen-4-chlor-anthranilsäure werden in 50 ml Isopropanol suspendiert und 222 mg (2,3 mmol) Schwefelsäure zugesetzt. Es wird 8,25 h unter Rückfluß erhitzt. Anschließend wird auf Raumtemperatur abgekühlt, das ausgefallene Produkt abgesaugt, mit Petrolether gewaschen und getrocknet. Man erhält 4,6 g (16,9 mmol; 78 %) N-Carbisopropoxymethylen-4-chlor-anthranilsäure.
Schmelzpunkt: 181°C
MS: 273, 271, 230, 228, 186, 184, 168, 166

### Beispiel 4:

Herstellung von N-Carbpropoxymethylen-4-chlor-anthranilsäure 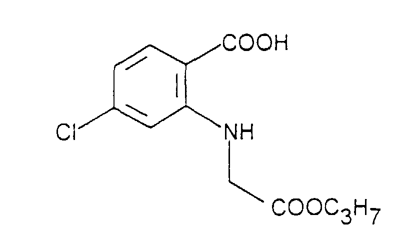 5 g (21,8 mmol) N-Carboxymethylen-4-chlor-anthranilsäure werden in 40 ml n-Propanol gelöst, mit 108 mg (1,05 mmol) konz. H₂SO₄ versetzt und 4,5 Stunden auf 70°C erhitzt. Anschließend wird auf Raumtemperatur abgekühlt, das ausgefallene Produkt abgesaugt und mit n-Propanol gewaschen. Nach Trocknen erhält man 3,6 g (13,3 mmol; 61 %) N-Carbpropoxymethylen-4-chlor-anthranilsäure als hellgelben Feststoff.
Schmelzpunkt: 155°C
Zur Aufnahme des Massenspektrums wird die noch freie Carboxylgruppe mit Diazomethan verestert, da sich N-Carbpropoxymethylen-4-chlor-anthranilsäure auf der GC-Säule zersetzt.

MS (N-Carbpropoxymethylen-4-chlor-anthranilsäuremethylester): 287, 285, 242, 200, 198, 168, 166.

### Beispiel 5

Herstellung von N-Carbbutoxymethylen-4-chlor-anthranilsäure 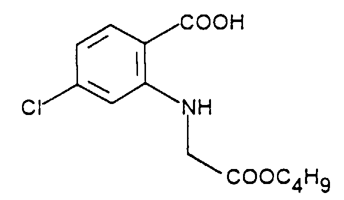 5 g (21,8 mmol) N-Carboxymethylen-4-chlor-anthranilsäure werden in 40 ml n-Butanol gelöst, 108 mg (1,05 mmol) konz. H₂SO₄ zugegeben und 2 Stunden auf 75°C erwärmt. Nach Abkühlen auf Raumtemperatur wird das ausgefallene Produkt abgesaugt, mit n-Butanol gewaschen und getrocknet. Man erhält 4,7 g (16,5 mmol; 76 %) hellgelbe kristalline N-Carbbutoxymethylen-4-chlor-anthranilsäure.
Schmelzpunkt: 130°C.

Zur Aufnahme des Massenspektrums wird die noch freie Carboxylgruppe mit Diazomethan verestert, da sich N-Carbbutoxymethylen-4-chlor-anthranilsäure auf der GC-Säule zersetzt.

MS (N-Carbbutoxymethylen-4-chlor-anthranilsäuremethylester): 301, 299, 242, 200, 198, 168, 166.

### Beispiel 6:

Herstellung von N-Carbhexoxymethylen-4-chlor-anthranilsäure 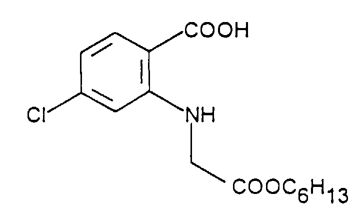 5 g (21,8 mmol) N-Carboxymethylen-4-chlor-anthranilsäure werden in 40 ml n-Hexanol gelöst, mit 108 mg (1,05 mmol) konz. H₂SO₄ versetzt und 2 Stunden auf 75°C erhitzt. Die Reaktionsmischung wird auf Raumtemperatur abgekühlt und das Produkt abgesaugt. Waschen mit Hexanol und Trocknen liefert 5,8 g (19,8 mmol; 91 %) Carbhexoxymethylen-4-chlor-anthranilsäure als hellgelben Feststoff.
Schmelzpunkt: 99°C.
Zur Aufnahme des Massenspektrums wird die noch freie Carboxylgruppe mit Diazomethan verestert, da sich N-Carbhexoxymethylen-4-chlor-anthranilsäure auf der GC-Säule zersetzt.

MS (N-Carbhexoxymethylen-4-chlor-anthranilsäuremethylester): 329, 327, 244, 242, 200, 198, 168, 166.

### Beispiel 7

Herstellung von N-Carbbenzoxymethylen-4-chlor-anthranilsäure 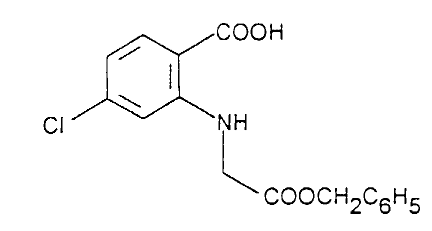 10 g (43,6 mmol) N-Carboxymethylen-4-chlor-anthranilsäure werden in 40 ml Benzylalkohol gelöst, 444 mg (4,3 mmol) H₂SO₄ zugesetzt und bei 110°C 2 Stunden gerührt. Man kühlt auf Raumtemperatur, saugt das ausgefallene Produkt ab, wäscht mit Benzylalkohol nach und trocknet. Man erhält 7,3 g (22,9 mmol; 53 %) hellgelbe kristalline N-Carbbenzoxymethylen-4-chlor-anthranilsäure.
Schmelzpunkt: 128°C.
Zur Aufnahme des Massenspektrums wird die noch freie Carboxylgruppe mit Diazomethan verestert, da sich N-Carbbenzoxymethylen-4-chlor-anthranilsäure auf der GC-Säule zersetzt.

MS (N-Carbbenzoxymethylen-4-chlor-anthranilsäuremethylester): 335, 333, 244, 242, 200, 198, 168, 166.

### Beispiel 8

### Herstellung von N-Carbethoxymethylen-4-chlor-anthranilsäure unter Azeotropdestillation von Wasser

230 g (1,0 mol) N-Carboxymethylen-4-chlor-anthranilsäure werden in 218 g Ethanol und 620 g Chloroform suspendiert und mit 11 g (107 mmol) konzentrierter Schwefelsäure versetzt. Zusätzlich wird die Chloroformmenge zugesetzt, die notwendig ist, einen mit dem Reaktionsgefäß verbundenen Wasserabscheider zu füllen. Es wird 7 Stunden unter Rückfluß erhitzt, wobei das bei der Veresterung entstehende Wasser kontinuierlich ausgekreist und im Wasserabscheider abgetrennt wird.
Anschließend wird das Chlorofom abdestilliert, auf Raumtemperatur abgekühlt, das auskristallisierte Produkt abgesaugt und mit Ethanol gewaschen.
Nach Trocknen erhält man 232 g (0,9 mol) N-Carbethoxymethylen-4-chloranthranilsäure.
Dies entspricht einer Ausbeute von 90 %.

### Vergleichsbeispiel 1 (Herstellung des Diethylesters)

Umsetzung von N-Carboxymethylen-4-chlor-anthranilsäure in Gegenwart einer großen Säuremenge als Katalysator (analog J. Heterocycl. Chem. 1987, 24, 812).

17,2 g (75 mmol) N-Carboxymethylen-4-chlor-anthranilsäure werden in 170 ml (134,16 g) Ethanol suspendiert und mit 40,3 g (390 mmol) konzentrierter Schwefelsäure innerhalb von 20 Minuten versetzt. Man erhitzt 24 Stunden zum Sieden.
Das Reaktionsgemisch enthält nach HPLC-Analyse 81 % des entsprechenden Diethylesters (N-Carbethoxymethylen-4-chlor-anthranilsäureethylester) und daneben 19 % eines nicht identifizierten Nebenproduktes.
N-Carbethoxymethylen-4-chlor-anthranilsäure (also der Monoester) kann nicht nachgewiesen werden.
Die Isolierung des Diesters erweist sich als schwierig, da man nach Abkühlen der Lösung, Abfiltrieren des ausgefallenen Produktes und Waschen desselben mit Ethanol lediglich 8 g des Diesters in Form einer zähen Masse, die sich nicht kristallisieren läßt, erhält. Weitere 2 g Produkt scheiden sich durch Aufgießen der Mutterlauge auf Wasser/Eis als Öl ab.

### Vergleichsbeispiel 2 (Herstellung des Dimethylesters)

Umsetzung von N-Carboxymethylen-4-chlor-anthranilsäure in Gegenwart einer großen Säuremenge als Katalysator (analog. J. Heterocycl. Chem. 1987, 24, 812).

17,2 g (75 mmol) N-Carboxymethylen-4-chlor-anthranilsäure werden in 170 ml Methanol suspendiert und mit 40,3 g (390 mmol) konzentrierter Schwefelsäure innerhalb von 20 Minuten versetzt. Man erhitzt 24 Stunden zum Sieden, läßt anschließend abkühlen und saugt das ausgefallene Produkt ab.

Nach Waschen mit Methanol und Trocknen erhält man 12,2 g (47 mmol) des entsprechenden Dimethylesters (N-Carbmethoxymethylen-4-chlor-anthranilsäuremethylester), entsprechend einer Ausbeute von 63 %. N-Carbmethoxymethylen-4-chlor-anthranilsäure (also der Monoester) kann nicht nachgewiesen werden.

### Beispiel 9

### Herstellung von N-Carbethoxymethylen-4-chlor-anthranilsäure unter Azeotropdestillation von Wasser

23 g (100 mmol) N-Carboxymethylen-4-chlor-anthranilsäure werden in 22 g Ethanol suspendiert und mit 1,1 g (10,7 mmol) konzentrierter Schwefelsäure versetzt. Außerdem setzt man 50 g 4-Methyl-2-pentanon zu und erhitzt 3 Stunden zum Sieden, wobei das bei der Veresterung entstehende Wasser kontinuierlich ausgekreist und in einem mit dem Reaktionsgefäß verbundenen Wasserabscheider abgetrennt wird.

Anschließend läßt man das Reaktionsgemisch auf Raumtemperatur abkühlen, filtriert das ausgefallene Produkt ab, wäscht es mit Ethanol und trocknet es. Man erhält 18,3 g (71 mmol) N-Carbethoxymethylen-4-chlor-anthranilsäure, entsprechend einer Ausbeute von 71 %.

## Patentansprüche

1. Verbindungen der Formel (I) 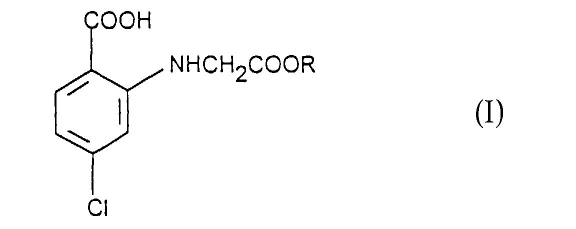 worin R für ein geradkettiges oder verzweigtes (C₁-C₂₀)-Alkyl, Phenyl oder CH₂-Phenyl, wobei die Alkylgruppe oder Phenylgruppe mit Halogen, (C₁-C₄)-Alkyl, (C₁-C₄)-Alkoxy substituiert sein kann, steht.

2. Verbindungen nach Anspruch 1, dadurch gekennzeichnet, daß in der Verbindung der Formel (I) R für ein geradkettiges oder verzweigtes (C₁-C₁₂)-Alkyl, Phenyl oder CH₂-Phenyl steht.

3. Verbindungen nach Anspruch 1, dadurch gekennzeichnet, daß in der Verbindung der Formel (I) R für ein geradkettiges oder verzweigtes (C₁-C₆)-Alkyl oder Phenyl steht.

4. N-Carbethoxymethylen-4-chloranthranilsäure, N-Carbmethoxymethylen-4-chloranthranilsäure, N-Carbisopropoxymethylen-4-chloranthranilsäure, N-Carbpropoxymethylen-4-chloranthranilsäure, N-Carbbutoxymethylen-4-chloranthranilsäure, N-Carbhexoxymethylen-4-chloranthranilsäure und N-Carbbenzoxymethylen-4-chloranthranilsäure.

5. Verfahren zur Herstellung der Verbindungen nach einem der Ansprüche 1 bis 4, dadurch gekennzeichnet, daß N-Carboxymethylen-4-chlor-anthranilsäure mit Alkoholen ROH, worin R die genannte Bedeutung besitzt, in Gegenwart eines Katalysators und gegebenenfalls eines Lösungsmittels, umgesetzt wird.

6. Verfahren nach Anspruch 5, dadurch gekennzeichnet, daß als Lösungsmittel aliphatische oder aromatische Kohlenwasserstoffe, insbesondere Cyclohexan, Toluol oder Benzol eingesetzt werden.

7. Verfahren nach Anspruch 5 oder 6, dadurch gekennzeichnet, daß der Alkohol ROH in einem Überschuß von 1 bis 1000 Mol, insbesondere 1,5 bis 500 Mol, bevorzugt 2 bis 200 Mol pro Mol N-Carboxymethylen-4-chlor-anthranilsäure eingesetzt wird.

8. Verfahren nach mindestens einem der Ansprüche 5 bis 7, dadurch gekennzeichnet, daß als Katalysator ein wasserentziehendes Mittel, eine starke anorganische Säure oder deren Salze oder eine Lewissäure eingesetzt werden.

9. Verfahren nach mindestens einem der Ansprüche 5 bis 8, dadurch gekennzeichnet, daß als Katalysator Dicyclohexylcarbodiimid, N,N-Carbonyldiimidazol, p-Toluolsulfonsäure, Pyridinium-p-toluolsulfonat, Trifluoressigsäure, Bortrifluorid, Natriumsulfat, Salzsäure oder Schwefelsäure, insbesondere Schwefelsäure oder Salzsäure, bevorzugt Schwefelsäure, eingesetzt werden.

10. Verfahren nach mindestens einem der Ansprüche 5 bis 9, dadurch gekennzeichnet, daß die Konzentration des Katalysators zwischen 0,5 und 100 Mol-%, insbesondere 1 bis 50 Mol-%, bevorzugt 2 bis 30 Mol-%,bezogen auf N-Carboxymethylen-4-chlor-anthranilsäure, beträgt.

11. Verfahren nach mindestens einem der Ansprüche 5 bis 10, dadurch gekennzeichnet, daß man die Umsetzung bei 20 bis 200, insbesondere 40 bis 160, bevorzugt 50 bis 120°C durchführt.

12. Verfahren nach mindestens einem der Ansprüche 5 bis 11, dadurch gekennzeichnet, daß die Reaktionstemperaturen zwischen 20°C und der Siedetemperatur des jeweiligen Alkohols ROH, insbesondere zwischen 40°C und der jeweiligen Siedetemperatur, bevorzugt zwischen 60°C und der jeweiligen Siedetemperatur, liegen.

13. Verfahren nach mindestens einem der Ansprüche 5 bis 12, dadurch gekennzeichnet, daß die Reaktionszeiten 0,5 bis 60 Stunden, insbesondere 0,75 bis 30 Stunden, bevorzugt 1 bis 20 Stunden betragen.

14. Verfahren nach mindestens einem der Ansprüche 5 bis 8, dadurch gekennzeichnet, daß man das bei der Veresterung entstehende Wasser durch Azeotropdestillation des Lösungsmittels abtrennt.

15. Verfahren nach mindestens einem der Ansprüche 5 bis 14, dadurch gekennzeichnet, daß man das bei der Veresterung entstehende Wasser durch Azeotropdestillation eines als Lösungsmittel eingesetzten einfach oder mehrfach chlorierten aliphatischen oder aromatischen Kohlenstoffwasserstoffs abtrennt.

16. Verfahren nach mindestens einem der Ansprüche 5 bis 15, dadurch gekennzeichnet, daß man das bei der Veresterung entstehende Wasser durch Zusatz eines Orthoesters entfernt.

## Claims

1. A compound of the formula (I) 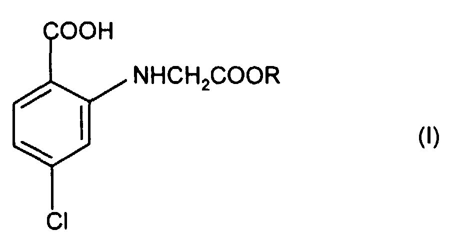 in which R is a straight-chain or branched (C₁-C₂₀)-alkyl, phenyl or CH₂-phenyl, where the alkyl group or phenyl group can be substituted by halogen, (C₁-C₄)-alkyl or (C₁-C₄)-alkoxy.

2. A compound as claimed in claim 1, in which, in the compound of the formula (I), R is a straight-chain or branched (C₁-C₁₂)-alkyl, phenyl or CH₂-phenyl.

3. A compound as claimed in claim 1, in which, in the compound of the formula (I), R is a straight-chain or branched (C₁-C₆)-alkyl or phenyl.

4. N-Carboethoxymethylene-4-chloroanthranilic acid, N-carbomethoxymethylene-4-chloroanthranilic acid, N-carboisopropoxymethylene-4-chloroanthranilic acid, N-carbopropoxymethylene-4-chloroanthranilic acid, N-carbobutoxymethylene-4-chloroanthranilic acid, N-carbohexoxymethylene-4-chloroanthranilic acid or N-carbobenzoxymethylene-4-chloroanthranilic acid.

5. A process for the preparation of a compound as claimed in one of claims 1 to 4, which comprises reacting N-carboxymethylene-4-chloro-anthranilic acid with an alcohol ROH, in which R has the meaning given, in the presence of a catalyst and, if appropriate, of a solvent.

6. The process as claimed in claim 5, wherein an aliphatic or aromatic hydrocarbon, in particular cyclohexane, toluene or benzene, is employed as the solvent.

7. The process as claimed in claim 5 or 6, wherein the alcohol ROH is employed in an excess of 1 to 1000 mol, in particular 1.5 to 500 mol, preferably 2 to 200 mol, per mole of N-carboxymethylene-4-chloro-anthranilic acid.

8. The process as claimed in at least one of claims 5 to 7, wherein a dehydrating agent, a strong inorganic acid or a salt thereof or a Lewis acid is employed as the catalyst.

9. The process as claimed in at least one of claims 5 to 8, wherein dicyclohexylcarbodiimide, N,N-carbonyldiimidazole, p-toluenesulfonic acid, pyridinium p-toluenesulfonate, trifluoroacetic acid, boron trifluoride, sodium sulfate, hydrochloric acid or sulfuric acid, in particular sulfuric acid or hydrochloric acid, preferably sulfuric acid, is employed as the catalyst.

10. The process as claimed in at least one of claims 5 to 9, wherein the concentration of the catalyst is between 0.5 and 100 mol%, in particular 1 to 50 mol%, preferably 2 to 30 mol%, based on N-carboxymethylene-4-chloro-anthranilic acid.

11. The process as claimed in at least one of claims 5 to 10, wherein the reaction is carried out at 20 to 200, in particular 40 to 160, preferably 50 to 120°C.

12. The process as claimed in at least one of claims 5 to 11, wherein the reaction temperature is between 20°C and the boiling point of the particular alcohol ROH, in particular between 40°C and the particular boiling point, preferably between 60°C and the particular boiling point.

13. The process as claimed in at least one of claims 5 to 12, wherein the reaction time is 0.5 to 60 hours, in particular 0.75 to 30 hours, preferably 1 to 20 hours.

14. The process as claimed in at least one of claims 5 to 8, wherein the water formed during the esterification is separated off by azeotropic distillation of the solvent.

15. The process as claimed in at least one of claims 5 to 14, wherein the water formed during the esterification is separated off by azeotropic distillation of a mono- or polychlorinated aliphatic or aromatic hydrocarbon employed as the solvent.

16. The process as claimed in at least one of claims 5 to 15, wherein the water formed during the esterification is removed by addition of an orthoester

## Revendications

1. Composés de formule (I) 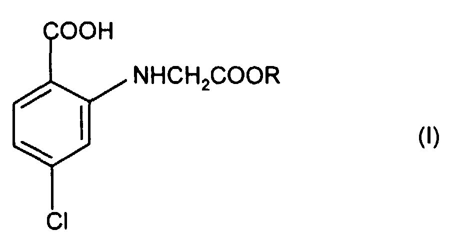 où R représente un groupe alkyle en C₁-C₂₀ à chaîne droite ou ramifiée, phényle ou CH₂-phényle, le groupe alkyle ou le groupe phényle pouvant être substitué par des substituants halogène, alkyle en C₁-C₄, alkoxy en C₁-C₄.

2. Composés selon la revendication 1, caractérisés en ce que dans les composés de formule (I), R représente un groupe alkyle en C₁-C₁₂ à chaîne droite ou ramifiée, phényle ou CH₂-phényle.

3. Composés selon la revendication 1, caractérisés en ce que dans les composés de formule (I), R représente un groupe alkyle en C₁-C₆ à chaîne droite ou ramifiée ou phényle.

4. Acide N-carbéthoxyméthylène-4-chloranthranilique, acide N-carbméthoxyméthylène-4-chloranthranilique, acide N-carbisopropoxyméthylène-4-chloranthranilique, acide N-carbpropoxyméthylène-4-chloranthranilique, acide N-carbbutoxyméthylène-4-chloranthranilique, acide N-carbhexoxyméthylène-4-chloranthranilique et acide N-carbbenzoxyméthylène-4-chloranthranilique.

5. Procédé de préparation des composés selon l'une des revendications 1 à 4, caractérisé en ce qu'on fait agir l'acide N-carboxyméthylène-4-chloranthranilique sur des alcools ROH, où R possède la signification citée, en présence d'un catalyseur et éventuellement d'un solvant.

6. Procédé selon la revendication 5, caractérisé en ce qu'on utilise, en tant que solvant, des hydrocarbures aliphatiques ou aromatiques, en particulier le cyclohexane, le toluène ou le benzène.

7. Procédé selon la revendication 5 ou 6, caractérisé en ce qu'on utilise l'alcool ROH en un excès de 1 à 1000 moles, en particulier de 1,5 à 500 moles, de préférence de 2 à 200 moles par mole d'acide N-carboxyméthylène-4-chloranthranilique.

8. Procédé selon au moins l'une des revendications 5 à 7, caractérisé en ce qu'on utilise en tant que catalyseur un agent déshydratant, un acide inorganique fort ou ses sels ou un acide de Lewis.

9. Procédé selon au moins l'une des revendications 5 à 8, caractérisé en ce qu'on utilise en tant que catalyseur le dicyclohexylcarbodiimide, le N,N-carbonyldiimidazole, l'acide p-toluène sulfonique, le p-toluène sulfonate de pyridinium, l'acide trifluoracétique, le trifluorure de bore, le sulfate de sodium, l'acide chlorhydrique ou l'acide sulfurique, en particulier l'acide sulfurique ou l'acide chlorhydrique, de préférence l'acide sulfurique.

10. Procédé selon au moins l'une des revendications 5 à 9, caractérisé en ce que la concentration en catalyseur s'élève de 0,5 à 100 % molaires, en particulier de 1 à 50 % molaires, de préférence de 2 à 30 % molaires, par rapport à l'acide N-carboxyméthylène-4-chloranthranilique.

11. Procédé selon au moins l'une des revendications 5 à 10, caractérisé en ce qu'on réalise la transformation à une température de 20 à 200, en particulier de 40 à 160, de préférence de 50 à 120°C.

12. Procédé selon au moins l'une des revendications 5 à 11, caractérisé en ce que les températures réactionnelles sont comprises entre 20°C et la température d'ébullition de l'alcool ROH respectif, en particulier entre 40°C et la température d'ébullition respective, de préférence entre 60°C et la température d'ébullition respective.

13. Procédé selon au moins l'une des revendications 5 à 12, caractérisé en ce que les durées réactionnelles sont de 0,5 à 60 heures, en particulier de 0,75 à 30 heures, de préférence de 1 à 20 heures.

14. Procédé selon au moins l'une des revendications 5 à 8, caractérisé en ce qu'on élimine l'eau formée au cours de l'estérification par distillation azéotropique du solvant.

15. Procédé selon au moins l'une des revendications 5 à 14, caractérisé en ce qu'on élimine l'eau formée au cours de l'estérification par distillation azéotropique d'un hydrocarbure aliphatique ou aromatique chloré une ou plusieurs fois utilisé comme solvant.

16. Procédé selon au moins l'une des revendications 5 à 15, caractérisé en ce qu'on élimine l'eau formée au cours de la distillation par ajout d'un orthoester.
